(19) Europäisches Patentamt

European Patent Office

Office européen des brevets

(11) Veröffentlichungsnummer: **0 315 222 B1**

## (12) EUROPÄISCHE PATENTSCHRIFT

(45) Veröffentlichungstag der Patentschrift: **19.11.92**

(21) Anmeldenummer: **88121701.2**

(22) Anmeldetag: **21.12.84**

(60) Veröffentlichungsnummer der früheren
Anmeldung nach Art. 76 EPÜ: **0 156 098**

(51) Int. Cl.⁵: **A61M  35/00, A61M 5/178**

(54) **Vorrichtung zur Applikation eines Gewebeklebstoffes.**

(30) Priorität: **29.03.84 AT 1063/84**

(43) Veröffentlichungstag der Anmeldung:
**10.05.89 Patentblatt  89/19**

(45) Bekanntmachung des Hinweises auf die
Patenterteilung:
**19.11.92 Patentblatt  92/47**

(84) Benannte Vertragsstaaten:
**AT BE CH DE FR GB IT LI LU NL SE**

(56) Entgegenhaltungen:
**EP-A- 0 037 393**
**DE-U- 8 119 976**
**US-A- 2 112 160**
**US-A- 4 406 656**

**GASTROENTEROLOGY Band 77, Nr. 4, Teil 1,**
**Oktober 1979, Seiten 642-646; W. G. LIN-**
**SCHEER et al.: "Control of Upper Gastrointe-**
**stinal Hemorrhage by Endoscopic Spraying**
**of Clotting Factors"**

(73) Patentinhaber: **IMMUNO Aktiengesellschaft**
**Industriestrasse 67**
**A-1221 Wien(AT)**

(72) Erfinder: **Redl, Heinz, Doz. Dr.**
**Windmühlgasse 7**
**A-1060 Wien(AU)**
Erfinder: **Habison, Georg, Dr.**
**Taubstummengasse 15/12**
**A-1040 Wien(AU)**

(74) Vertreter: **Wolfram, Gustav, Dipl.-Ing.**
**Patentanwälte Sonn, Pawloy, Weinzinger &**
**Wolfram, Riemergasse 14**
**A-1010 Wien(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Vorrichtung zur Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u.dgl., welcher Gewebeklebstoff durch Zusammenbringen von Lösungen der Proteine und blutgerinnungsfördernder Gerinnungsfaktoren in situ gebildet wird, mit einer Mehrzahl von in Anschlußstücken endigenden Behältern für die Aufnahme der zu applizierenden Komponenten, vorteilhaft mit einer Mehrzahl von genormten, in Konussen endigenden Einwegspritzenkörpern aus Kunststoffmaterial, wobei auf die Anschlußstücke der Behälter ein Verbindungskopf aufsetzbar ist, der für jede der zu applizierenden Komponenten einen eigenen zur Stirnseite des Verbindungskopfes führenden Förderkanal aufweist.

Eine bekannte Vorrichtung dieser Art ist in der AT-B - 366.916 beschrieben. Als Komponenten des Gewebeklebstoffes können einerseits eine Faktor XIII und Fibrinogen enthaltende Lösung und andererseits eine Thrombin enthaltende Lösung verwendet werden. Diese Komponenten werden in einer auf den Verbindungskopf aufgesetzten Mischkanüle vermischt und auf die zu behandelnde bzw. zu schützende Wundstelle aufgebracht. Diese Vorrichtung kommt in erster Linie für solche Anwendungen in Betracht, bei welchen die Applikationsstelle gut zugänglich und leicht erreichbar ist.

Aus einer Veröffentlichung von Linscheer und Fazio in Gastroenterology 77, 642-649 (1979) ist auch eine Einrichtung zur Auftragung von gerinnbaren Substanzen in Körperhöhlen bekannt geworden, welche Einrichtung einen plastischen Katheter aufweist. Die Einrichtung umfaßt eine Mehrzahl von Behältern für die zu mischenden Komponenten, an die Schlauchleitungen angesetzt sind, die mit einem mehrlumigen Katheter untrennbar verbunden sind. Eine solche Einrichtung hat jedoch den Nachteil, daß sie nur in Verbindung mit einem Endoskop verwendbar ist, mit dessen Hilfe es an die Applikationsstelle geführt werden muß.

Schließlich ist aus der US-PS 4,406,656 ein kollapsibler Venenkatheter bekannt, welcher mehrere Lumina für medizinische Infusionsflüssigkeiten aufweist, und in einem Lumen ein Formdraht untergebracht ist, der ein Knicken verhindert. Dennoch muß der Katheter so flexibel sein, daß er dem Verlauf einer Vene folgen kann, ohne deren Wand zu verletzen.

Der Erfindung liegt die Aufgabe zugrunde, eine Vorrichtung der eingangs bezeichneten Art zu schaffen, mit der die Komponenten des Gewebeklebstoffes auch an schwer zugängliche Applikationsstellen herangebracht werden können, ohne daß eine vorzeitige Vermischung der Komponenten stattfindet und ohne ein Endoskop zu Hilfe nehmen zu müssen. Mit der Vorrichtung soll wahlweise ein flüssiger Austritt der Komponenten an der Mündung des Katheters oder eine Zerstäubung der Komponenten erreicht werden können.

Diese Aufgaben der Erfindung werden bei einer Vorrichtung der eingangs bezeichneten Art dadurch gelöst, daß der Verbindungskopf einen zusätzlichen Förderkanal für ein medizinisches Gas besitzt, der eng benachbart zu den übrigen Förderkanälen zur Stirnseite des Verbindungskopfes führt, und daß auf den Verbindungskopf ein mehrlumiger Katheter in fluchtender Verbindung der Förderkanäle aufsteckbar ist, der außer den Lumen für jede der zu applizierenden Komponenten und für das medizinische Gas noch ein zusätzliches Lumen für einen Formdraht besitzt.

Die erfindungsgemäße Vorrichtung stellt sicher, daß es zu keiner vorzeitigen Mischung des Gewebeklebstoffes und damit zu keiner Verstopfung kommt. Die Vorrichtung ist in weitem Umfang einsetzbar, sowohl für kleinflächige als auch für großflächige Applikationsbereiche, wobei je nach Wahl der Fördergeschwindigkeit und der Fördermenge des medizinischen Gases an der Mündung ein flüssiger Austritt der Komponenten bzw. eine Zerstäubung erreicht wird. Mit Hilfe des Formdrahtes ist der Katheter plastisch verformbar und behält die ihm gegebene Form bei.

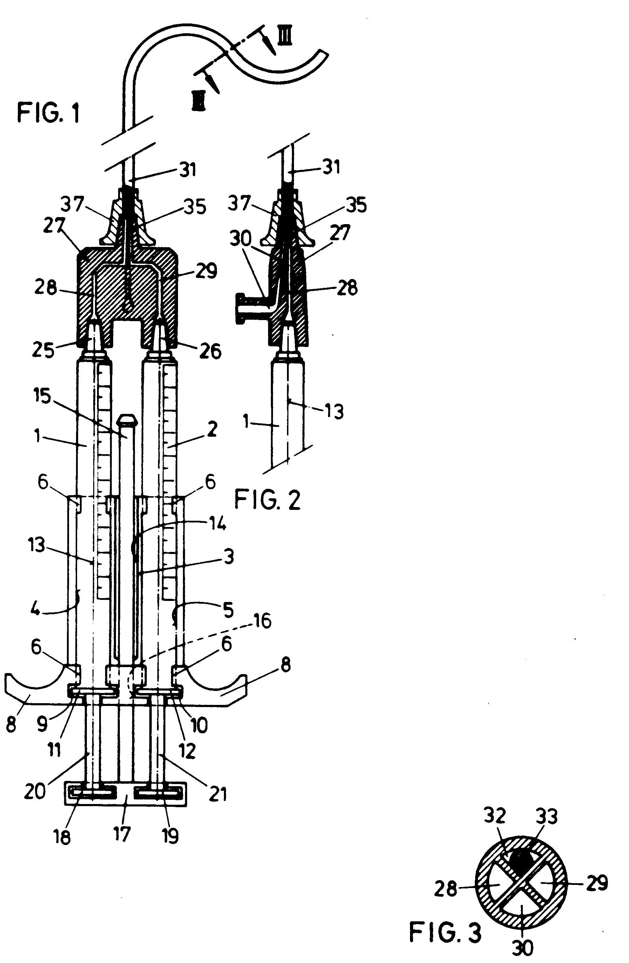

Die erfindungsgemäße Vorrichtung ist an einem Ausführungsbeispiel in der Zeichnung näher erläutert, wobei Fig. 1 eine teilweise geschnittene Seitenansicht der Vorrichtung mit aufsetzbarem Katheter, Fig. 2 einen Teilschnitt der Vorrichtung und Fig. 3 einen Querschnitt durch den Katheter gemäß der Linie III-III der Fig. 1 zeigen.

Mit 1 und 2 sind zwei genormte Einwegspritzenkörper bezeichnet, von denen einer zur Aufnahme einer Thrombin enthaltenden Lösung und der zweite zur Aufnahme einer Faktor XIII und Fibrinogen enthaltenden Lösung dient. Die Spritzenkörper 1, 2 sind zweckmäßig als genormte Einwegspritzenkörper aus Kunststoff ausgebildet. Sie sind gemeinsam in eine Halteeinrichtung 3 eingesetzt. Diese weist zwei U-förmig gestaltete Rinnen 4, 5 auf, die jeweils an ihren Enden mit Noppen 6 ausgestattet sind, so daß die Spritzenkörper 1, 2, die von oben in die Rinnen eingesetzt werden, einrasten und durch die Noppen festgehalten werden.

Am Ende der Halteeinrichtung sind Fingergriffe 8 vorgesehen, die U-förmig gestaltete Erweiterungen 9, 10 aufweisen, in die die Flanschenden 11, 12 der Spritzenkörper ragen, so daß die Spritzenkörper in Richtung ihrer jeweiligen Längsachse 13 fixiert sind.

Zwischen den U-förmigen Rinnen 4, 5 ist eine Ausnehmung 14 für eine Führungsstange 15 vorgesehen, die im Bereich der Fingergriffe 8 die Bohrung 16 der Halteeinrichtung durchsetzt. Die Führungsstange kann mit einer gemeinsamen Betätigungseinrichtung 17 für die Daumenaufsätze 18, 19 der Kolben 20, 21 verbunden sein. Diese Kolben können aber auch jeweils für sich betätigt werden.

Die beiden Konusse 25, 26 der Spritzenkörper ragen in entsprechend geformte Ausnehmungen des Verbindungskopfes 27 und sind mit diesem verbunden. Innerhalb des Verbindungskopfes führt von jedem Einsteckkonus 25, 26 ein eigener Förderkanal 28, 29 an die Stirnseite des Verbindungskopfes. Außerdem ist im Verbindungskopf ein weiterer Förderkanal 30 für ein medizinisches Gas vorgesehen, der ebenfalls an die Stirnseite des Verbindungskopfes führt, parallel und eng benachbart mit den Förderkanälen 28 und 29.

Der Verbindungskopf 27 weist einen sockelförmigen Fortsatz 35 auf, in dem die drei parallel laufenden Förderkanäle 28, 29 und 30 vereinigt sind. Auf diesen Fortsatz 35 ist ein vierlumiger Katheter 31 (Fig. 3) in fluchtender Verbindung der Förderkanäle aufsteckbar, wobei ein Lumen die Fortsetzung des Förderkanales 28, ein anderes Lumen die Fortsetzung des Förderkanales 29 ist, ein drittes Lumen dem Luftförderkanal 30 entspricht und im vierten Lumen 32 ein Formdraht 33 eingebracht ist.

Durch Druck auf die Kolben 18, 19 bzw. die Betätigungsvorrichtung 17 werden die zu vermischenden Komponenten der Applikationsstelle zugeführt.

## Patentansprüche

1. Vorrichtung zur Applikation eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung u.dgl., welcher Gewebeklebstoff durch Zusammenbringen von Lösungen der Proteine und blutgerinnungsfördernder Gerinnungsfaktoren in situ gebildet wird, mit einer Mehrzahl von in Anschlußstükken (25, 26) endigenden Behältern (1, 2) für die Aufnahme der zu applizierenden Komponenten, vorteilhaft mit einer Mehrzahl von genormten, in Konussen endigenden Einwegspritzenkörpern aus Kunststoffmaterial, wobei auf die Anschlußstücke (25, 26) der Behälter (1, 2) ein Verbindungskopf (27) aufsetzbar ist, der für jede der zu applizierenden Komponenten einen eigenen zur Stirnseite des Verbindungskopfes führenden Förderkanal (28, 29) aufweist, dadurch gekennzeichnet, daß der Verbindungskopf (27) einen zusätzlichen Förderkanal (30) für ein medizinisches Gas besitzt, der eng benachbart zu den übrigen Förderkanälen zur Stirnseite des Verbindungskopfes (27) führt, und daß auf den Verbindungskopf (27) ein mehrlumiger Katheter (31) in fluchtender Verbindung der Förderkanäle aufsteckbar ist, der außer den Lumen (28, 29, 30) für jede der zu applizierenden Komponenten und für das medizinische Gas noch ein zusätzliches Lumen (32) für einen Formdraht (33) besitzt.

## Claims

1. An arrangement for applying a tissue adhesive based on human or animal proteins to seamlessly or seam-supportingly connect human or animal tissue or organ parts, to seal wounds, stop bleedings and the like, which tissue adhesive is formed in situ by combining solutions of the proteins and of blood-clot-promoting coagulation factors, comprising a plurality of containers (1, 2) ending in joining pieces (25, 26) and adapted to receive the components to be applied, advantageously a plurality of standardized disposable syringe bodies of synthetic material ending in coni, wherein a connecting head (27) is attachable to the joining pieces (25, 26) of the containers (1, 2), which includes a separate conveying channel (28, 29) for each of the components to be applied, leading to the front side of the connecting head, characterized in that the connecting head (27) includes an additional conveying channel (30) for a medicinal gas, which leads to the front side of the connecting head (27) closely adjacent to the other conveying channels, and that a multilumen catheter (31) is attachable to the connecting head (27) in aligning connection with the conveying channels, which includes an additional lumen (32) for a shaping wire (33) in addition to the lumina (28, 29, 30) for each of the components to be applied and for the medicinal gas.

## Revendications

1. Dispositif d'application d'une colle pour tissus à base de protéines humaines ou animales, pour relier, sans couture ou en appui à une couture, des parties de tissu ou d'organe humain ou animal, pour cicatriser une blessure, pour arrêter le sang et analogues, colle pour tissu qui est formée in situ par la réunion de solutions de protéines et de facteurs coagulants favorisant la coagulation du sang, avec une pluralité de récipients (1, 2), qui se terminent dans des éléments de raccord (25, 26), pour recevoir les composants à appliquer, de préférence avec une pluralité de seringues d'injection normalisées, en matériau plastique, se terminant en cône, dispositif dans le cas duquel on peut enficher, sur les éléments de raccord (25, 26) des récipients (1, 2), une tête de liaison (27) qui présente, pour chacun des composants à appliquer, son propre canal de transport (28, 29) conduisant à la face frontale de la tête de liaison, dispositif caractérisé par le fait que la tête de liaison (27) possède, pour un gaz médicinal, un canal de transport supplémentaire (30) qui, étroitement voisin des autres canaux de transport, conduit à la face frontale de la tête de liaison (27), et par le fait que sur la tête de liaison (27), on peut enficher, en liaison d'alignement avec les canaux de transport, un cathéter (31) à plusieurs passages, qui, en dehors des passages (28, 29, 30) pour chacun des composants à appliquer et pour le gaz médicinal, possède encore un passage supplémentaire (32) pour un fil profilé (33).

FIG. 1

FIG. 2

FIG. 3